## Europäisches Patentamt

## European Patent Office

## Office européen des brevets

(11) Publication number: **0 208 472**
**B1**

## EUROPEAN PATENT SPECIFICATION

(45) Date of publication of patent specification: **06.02.91**

(21) Application number: **86304951.6**

(22) Date of filing: **26.06.86**

(51) Int. Cl.⁵: **C 12 N 15/56,** C 12 N 9/36,
C 12 N 15/81

(54) Microbiological process for production of human lysozyme.

(30) Priority: **26.06.85 JP 139710/85**

(43) Date of publication of application:
**14.01.87 Bulletin 87/03**

(45) Publication of the grant of the patent:
**06.02.91 Bulletin 91/06**

(84) Designated Contracting States:
**AT BE CH DE FR GB IT LI NL SE**

(56) References cited:
**EP-A-0 155 189**

(73) Proprietor: **SUMITOMO CHEMICAL COMPANY, LIMITED**
Kitahama 4-chome 5-33
Chuo-ku Osaka 541 (JP)
(73) Proprietor: **Director-General of the Agency of Industrial Science and Technology**
Ministry of International Trade & Industry 3-1
Kasumigaseki 1-chome
Chiyoda-ku Tokyo 100 (JP)

(72) Inventor: **Jigami, Yoshifumi**
6-255, Ushikumachi-Sakaemachi
Inashiki-gun Ibaraki 300-07 (JP)
Inventor: **Uemura, Hiroshi**
603-809, Sakuramura-Azuma 1-chome
Niihari-gun Ibaraki 305 (JP)
Inventor: **Muraki, Michirou**
417-204, Yatabemachi-Matsushiro 4-chome
Tsukuba-gun Ibaraki 300-32 (JP)
Inventor: **Tanaka, Hideaki**
810-108, Sakuramura-Azuma 2-chome
Niihari-gun Ibaraki 305 (JP)
Inventor: **Nakazato, Satoshi**
407-29, Toyoshiki
Kashiwa-shi Chiba 277 (JP)
Inventor: **Kishimoto, Fumitaka**
4-8-1, Daiwahigashi
Kawanishi-shi Hyogo 666-01 (JP)

**EP 0 208 472 B1**

⁷² Inventor: **Agui, Hideo**
**5-5-203, Satsukigaoka 4-chome**
**Ikeda-shi Osaka 563 (JP)**
Inventor: **Ogino, Shigeo**
**5-30-202, Koshikiiwamachi**
**Nishinomiya-shi Hyogo 662 (JP)**

⁷⁴ Representative: **Myerscough, Philip Boyd et al**
**J.A.Kemp & Co. 14, South Square Gray's Inn**
**London, WC1R 5EU (GB)**

**Description**

The present invention relates to a microbiological process for producing human lysozyme utilizing recombinant DNA technology.

Lysozyme is an enzyme found in tears, saliva, nasal mucus, milk, lymphatic gland, leucocyte, serum, cartilage, lung, kidney, spleen, liver, intestinal canal, parotid gland, skin or sperm and also in urine of leukemia patients. It has the enzymatic activity, as muramidase, to hydrolyze the $\beta$-1,4-linkage between N-acetylglucosamine and N-acetylmuramine.

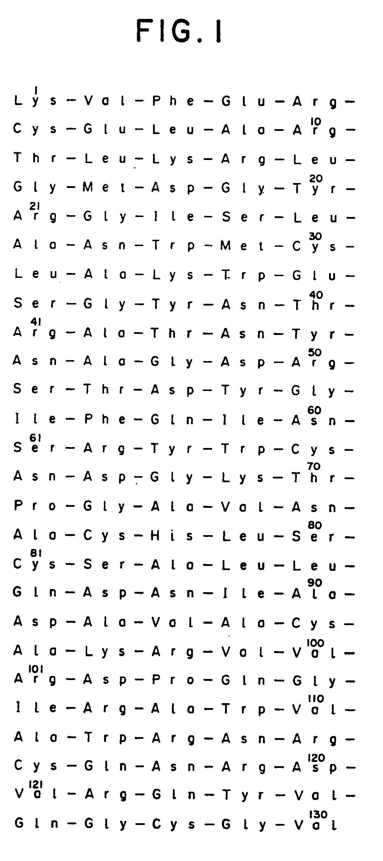

It has been reported that the lysozyme derived from human milk consists of 130 amino acids linked in the sequence given in Fig. 1 (see, for example, Funatu et al. "YŌKIN KŌSO" p. 55—58, KŌDANSHA SCIENTIFIC. JAPAN (1977)).

Lysozyme can be used, alone or in combination with antibiotics, as a preservative for foods or an antimicrobial agent because of its bacteriolytic activity. It can also be used for the treatment of various diseases because of its blood-coagulating activity, haemostatic activity, anti-inflamatory activity and apophlegamic activity.

Lysozyme can be isolated from human milk, urine or other human sources, but such methods are not feasible for the large scale production of human lysozyme because it is difficult to collect the human raw material and hence it can not meet even the demands for its therapeutic uses.

The present invention provides recombinant DNA technology which will enable the large scale production of human lysozyme in a purified form at reasonable costs.

In principle, a process for producing human lysozyme utilizing a chemically synthesized human lysozyme gene involves the following steps:

(1) Design of the gene

(2) Chemical synthesis of the gene

(3) Insertion of the gene into a suitable expression vector plasmid

(4) Transformation of a suitable host with the resulting recombinant vector plasmid

(5) Production of human lysozyme by cultivation of the resulting transformant and is isolation of the human lysozyme from the cultured transformant.

The present inventors have already provided a process for producing human lysozyme utilizing *Escherichia coli* as hosts in the above mentioned procedures.

Yeasts, *Saccharomyces cerevisiae* have many advantages over *E. coli* as the hosts to be used for the production of biologically active substances by the recombinant DNA technology.

For example,

Cultivation of yeasts is safer and easier than that of *E. coli* and yeasts can be cultured at a higher density than *E. coli*.

The safety of the products is higher because possible contamination can more easily be prevented.

It is more likely that expression products of genes of the higher animals by yeasts are produced in their right structures or natural forms.

Yeasts can stably be used for production of lysozyme because they are insensitive to lysozyme.

However, yeasts have some disadvantages as the hosts for foreign genes. It is known that, in order for foreign genes to be effectively expressed, the genes must be inserted at a right place into the DNA region controlling the transcription and translation (hereinafter referred to as "5'-noncoding region") of an expression vector plasmid, but desirous DNA structure (DNA sequences) of the 5'-noncoding region for yeasts has not yet made clear to the same extent as in the case of *E. coli*. Accordingly. it is very difficult at the present time to design a suitable DNA structure of the 5'-noncoding region of an expression vector plasmid for a particular foreign gene for yeasts. It can only be discovered through extensive trial and error type of studies.

Under the circumstances, the present inventors have extensively studied on the production of human lysozyme utilizing transformant yeasts and accomplished the present invention. According to the present invention, lysozyme is produced at a high expression level.

Description of the Drawing

Fig. 1 shows the amino acid sequence of human lysozyme.

Fig. 2 shows the DNA sequence of the synthetic structural gene coding for human lysozyme.

Fig. 3 shows the DNA sequence of the synthetic human lysozyme gene wherein the translation initiation and stop codons are attached to the structural gene and restriction sites are added to the 5'- and 3'- ends of the gene.

Fig. 4 shows a set of DNA fragments constituting the synthetic human lysozyme gene and how they are assembled into the entire human lysozyme gene.

Each fragment is indicated with an arrow and given an identification code, i.e., HLY-1 through HLY-56. Intermediate block fragments are indicated with longer arrows and given identification codes, i.e A to I.

Fig. 5 is a schematic view indicating the construction of the recombinant plasmids YEpHLY-1 and YEpHLY-2.

Fig. 6 is a schematic view indicating the manner of the ligation between Gal 10 promotor region and the human lysozyme gene.

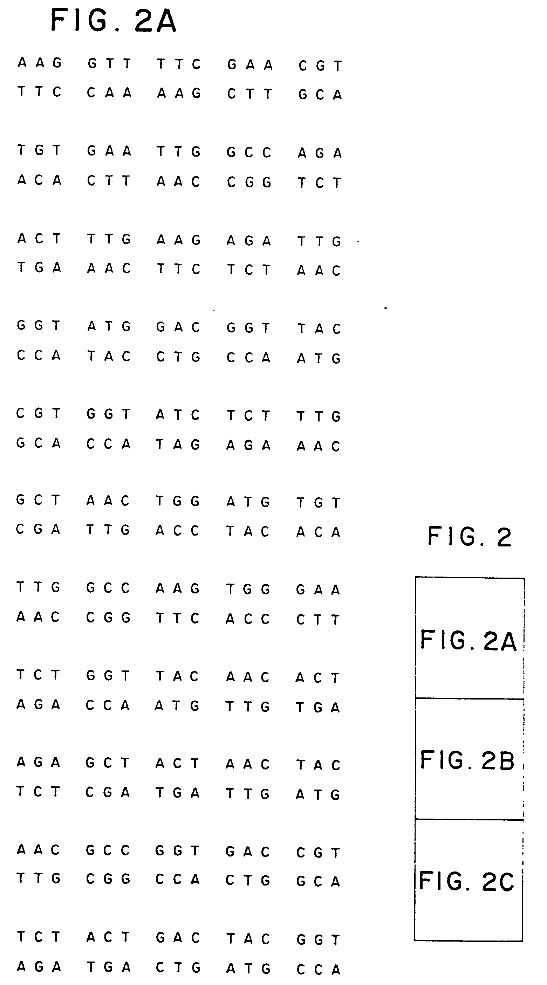

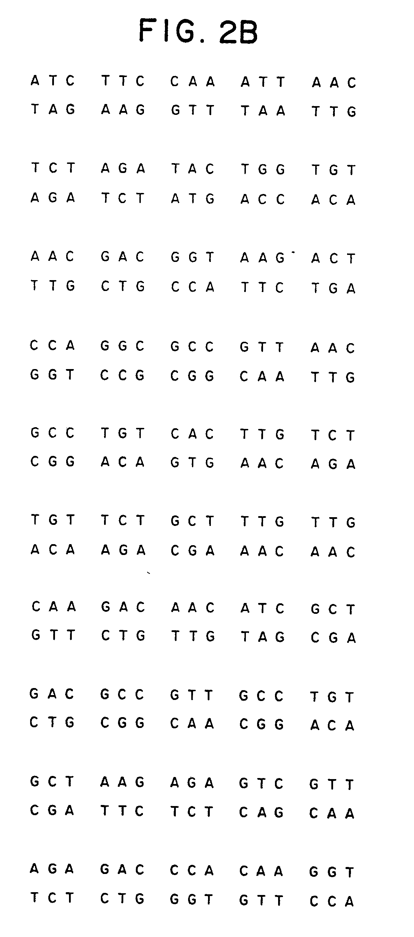

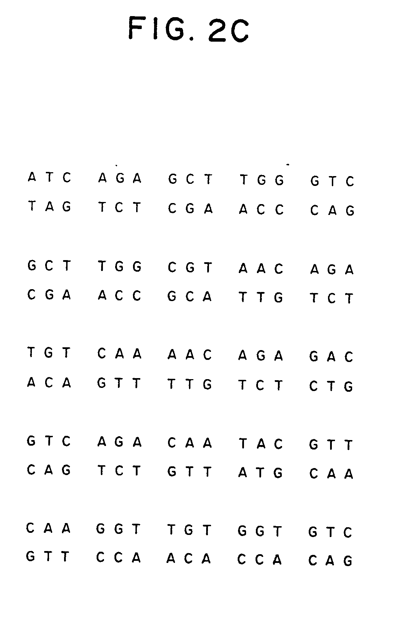

The present invention provides an expression vector plasmid for yeasts comprising a yeast expression vector DNA containing GAL 10 promoter and a synthetic human lysozyme gene having the DNA sequence given in Fig. 2 inserted into said yeast expression vector at its insertion site and linked to said promoter through a 5'-noncoding region containing a DNA sequence which is

AAGAATTTTTGAGGAGTTTAAT  or  TTTTGAGTCGAGATCCGTTAGG

TTCTTAAAAACTCCTCAAATTA      AAAACTCAGCTCTAGGCAATCC

The invention also provides transformant yeasts capable of producing human lysozyme produced by transforming yeast with an expression vector plasmid of the invention and a process for producing human lysozyme which comprises culturing the transformant yeasts capable of producing human lysozyme in a suitable culture medium and collecting the human lysozyme from the culture transformant yeast.

According to the present invention a human lysozyme gene can be prepared by the following procedures:

(1) Design of the gene
    1) Structural gene
    The DNA sequence coding for human lysozyme which satisfies the following conditions is first designed:
    (A) As many as possible preferential codon usages for yeasts are used.
    (B) Between or within the fragments which compose the gene, there should not be undesirable, complementary sequences.

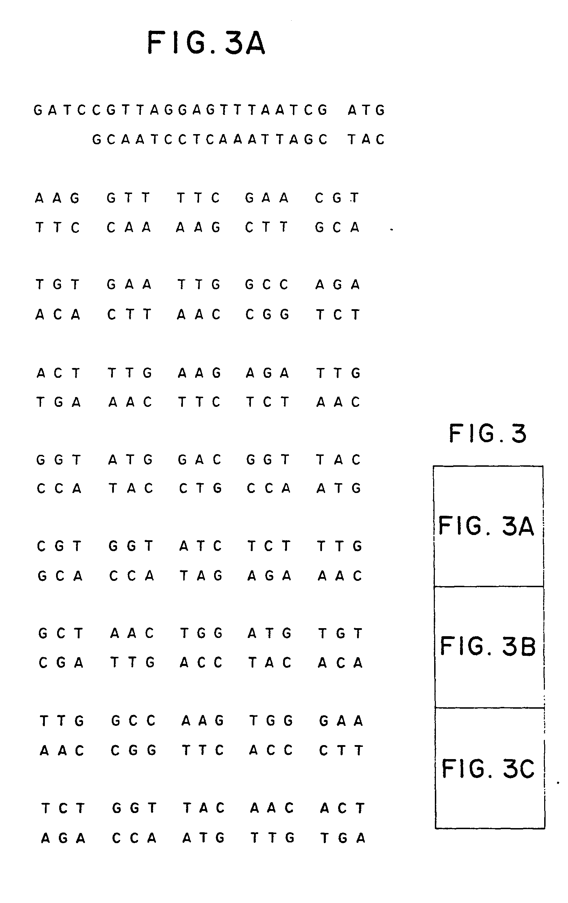

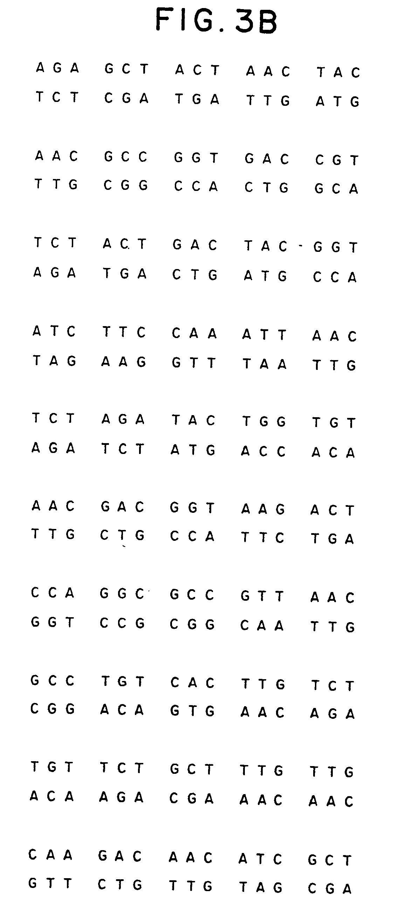

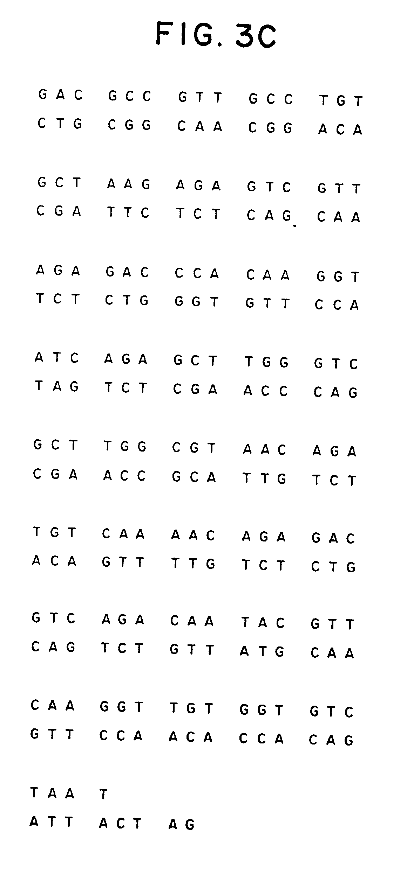

    2) The whole gene
    It is desirable for the whole gene to have the following arrangements in order to be easily used:
    (A) In order to have translation initiated and terminated, translation initiation codon as well as stop codon should be added to the 5'- and 3'- ends of the structural gene.
    (B) In order for the gene to be easily inserted into and cleaved off a plasmid, there should be restriction sites at 5'- and 3'- ends of the gene.
    (C) In order to make the selection of the transformants easier, one or more restriction sites should be incorporated within the gene. A structural gene designed as above is shown in Fig 2.
    By inserting the structural gene of the Fig. 2 into a suitable expression vector, a recombinant plasmid which enables the expression of the gene by yeasts can be constructed. In Fig. 3, there is given an example of a gene sequence wherein the translation initiation and termination codons are attached to the structural gene of Fig. 2 and restriction sites are added to 5'- and 3'- terminals of the gene. Because this synthetic human lysozyme gene has both the translation initiation and termination codons, there is less limitation in selecting expression vector plasmids to be used. It is also possible to insert this gene into an adequate vector after cleaving it with a suitable restriction enzyme (e.g., Cla I).

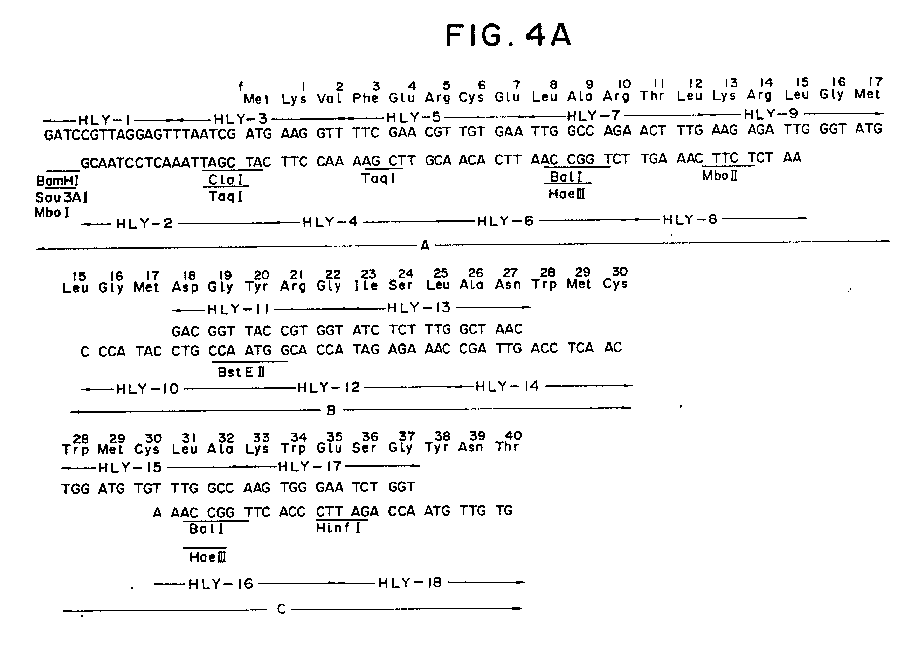

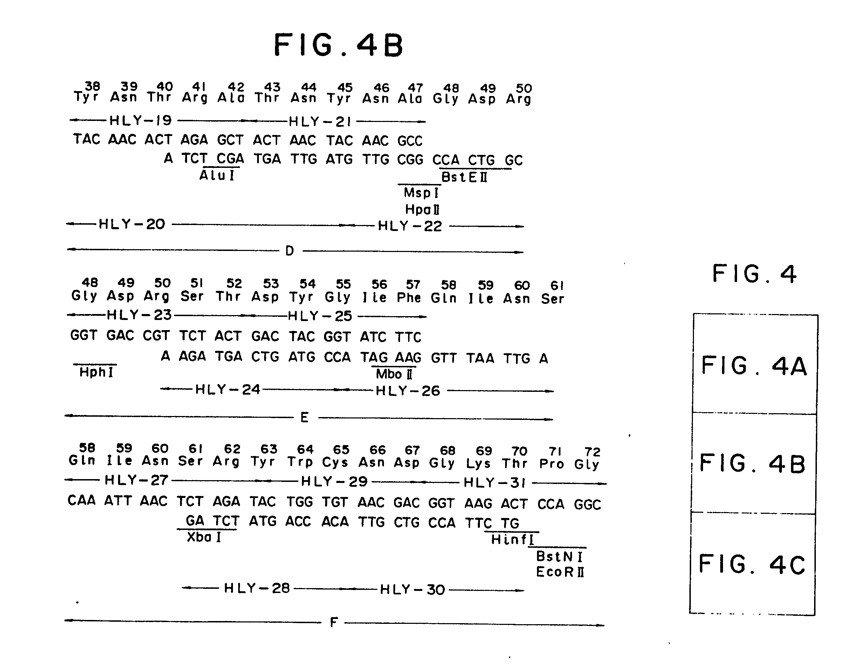

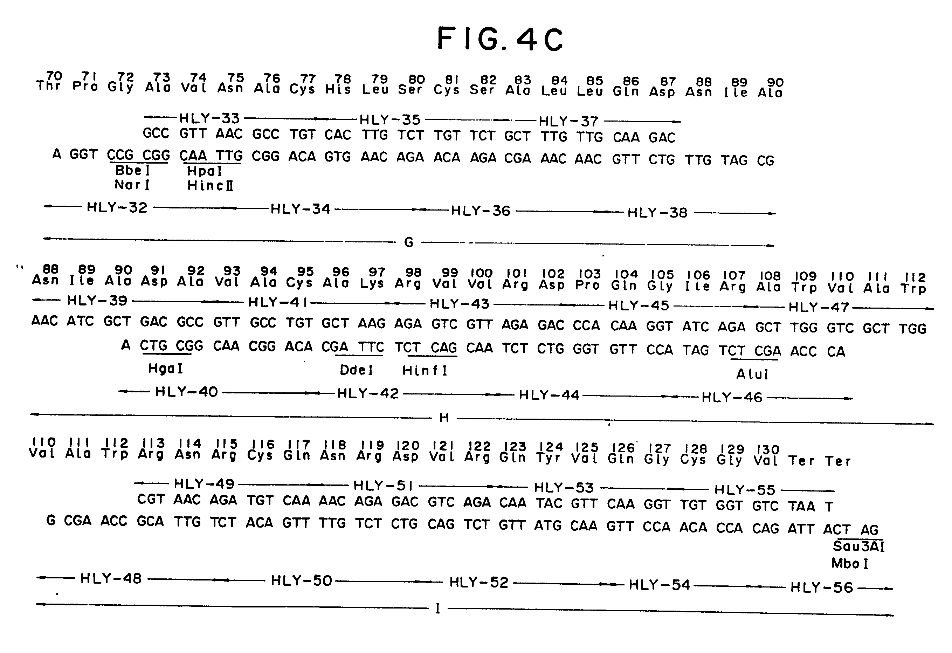

(2) Synthesis of the gene
    For the construction of the gene designed as above, an adequate number of DNA fragments which constitute (+) or (−) strands of the gene are first synthesized and the fragments are then assembled into the gene. It is preferable that each fragment contains 11—19 bases and each set of duplexes has at least 6 complementary base sequences.
    One preferred arrangement for the synthesis of the gene is given in Fig. 4. The chemical synthesis and purification of the fragments, phosphorylation of hydroxyl groups at the 5'- ends and ligation of the fragments are carried out according to conventional methods.

(3) Construction of a recombinant plasmid
    1) Insertion of the human lysozyme gene into an expression vector plasmid
    The human lysozyme gene synthesized as above is then inserted, at an adequate insertion site, into either plasmids autonomously replicable in host cells or expression vector plasmids which are autonomously replicable and which are capable of expressing an inserted foreign gene in host cells. The insertion of the gene into plasmids can be carried out by the methods conventionally used in the field of molecular biology. Amplification of the human lysozyme gene of the present invention can be carried out using known vector plasmids such as pBR322, pAT153 (see, for example, Twigg et al. Nature, 283, 216—218 (1980)). Isolation of the plasmid DNA can also be carried out by a conventional method used in the field of molecular biology.
    Expression of the human lysozyme gene of the present invention is performed using various kinds of known plasmids having GAL10 promoter (Broach et al. Experimental Manipulation of Gene Expression 83—117 (1983)).
    Among such vector plasmids, YEp51 (Broach et al. Experimental Manipulation of Gene Expression, 83—117 (1983)) is a particularly preferred vector plasmid to be used for expression of the human lysozyme gene of the present invention.

2) Determination of the direction of the gene

The direction of the gene inserted into a plasmid can be determined by cleaving the plasmid having the gene at a restriction enzyme recognition site located within the gene and also at other recognition site outside the gene and then analyzing the resulting DNA fragments by agarose gel electrophoresis according to a method conventionally used in the field of the present invention.

(3) Transformation

1) Hosts

For the preparation and amplification of recombinant plasmids containing the human lysozyme gene, E. coli, for example, E. coli C600 (Nelson et al. Virology 108 338—350 (1981) can be used.

According to the present invention, the yeasts transformed with the expression vector plasmids containing the synthetic human lysozyme gene are used in producing human lysozyme. A preferred example of yeasts as the host cells for this purpose is Saccharomyces cerevisiae, particularly S. cerevisiae KK4 strain (Nogi et al. Mol. Gen. Genet., 195, 29—34 (1984)). Other types of known S. cerevisiae yeasts can also be used as the host cells in producing the transformant yeasts of the present invention. For example, those listed in catalogues issued by Yeast Genetic Stock Center or some other public depositories such as American Type Culture Collection. These yeasts are available from such depositories.

2) Transformation

The transformation operation per se is conducted according to the procedures conventionally used in the field of molecular biology. For example, the transformation procedures for E. coli are disclosed by Cohen et al. in Proc. Natl. Acad. Sci. U.S.A., 69 2110—2114 (1972) and by Maniatis et al in Molecular Cloning, 249—253 (1982); and for yeasts by Hinnen et al. Proc. Natl. Acd. Sci. U.S.A., 75 1929 (1978) and by Ito et al. in J. Bacteriol., 153 163—168 (1983).

3) Transformants

Preferred examples of the transformant yeasts are S. cerevisiae KK4 (YEpHLY-1) and S. cerevisiae KK4 (YEpHLY-2), which are obtained by the transformation of S. cerevisiae KK4 with the recombinant plasmids YEpHLY-1 and YEpHLY-2, respectively.

(4) Production of human lysozyme

Human lysozyme can be produced by cultivating the transformant yeasts obtained as above in a suitable culture medium in the manner conventionally used in the fields of molecular biology and fermentation. The resulting human lysozyme can be detected and quantitatively analyzed by the immunoprecipitation method, radioimmunoassay (Yuzuriha et al. Chem. Pharm. Bull., 27 2802—2806 (1979); ibid., 26 908—914 (1978)), or enzymatic activity assay (Sidhan et al. Agric. Biol. Chem., 45 1817—1823 (1981)) and Morsky. Anal. Biochem., 128 77—85 (1983)). Thus prepared human lysozyme can be purified and recovered by using one or more methods usually used in the fields of biochemistry or fermentation.

The following examples are given to illustrate the present invention and not intended to limit the scope of the invention in any manner.

Example 1

Preparation of synthetic human lysozyme gene

For the construction of the human lysozyme gene, the 56 oligonucleotides shown in Fig. 4 were synthesized by the solid phase synthetic method in the manner known per se (Itakura et al. Nuclei. Acids Res., 10 1755 (1982)). Commercially available polystyrene resin having a crosslinking rate of 1%. to which nucleotides were bound are used. Completely protected dinucleotides were either purchased from Nippon Zeon Co., Ltd., or YAMASA SHOYU CO., LTD. or prepared by a known method (Gait et al. Nucleic Acids Res., 9 1691 (1981)).

The following detailed disclosure of the synthesis of a pentadecanucleotide: Gp Ap Tp Cp Cp Gp Tp Tp Ap Gp Gp Ap Gp Tp T is given as a representative example of the synthesis of the oligonucleotides constituting the synthetic human lysozyme:

With 1 ml of dichloromethane-isopropanol mixed solvent (85: 15, V/V) containing 1 M zinc bromide, 40 mg of polystyrene resin binding completely protected thymidine (4 µmol) through linkers was treated for 30 seconds for 6 or 7 times until red color due to dimethoxytrityl cation disappeared, thereby removing dimethoxytrityl, the protective group for the 5'-hydroxyl.

Completely protected dinucleotide; GpTp (25 mg) was treated with triethylamine-pyridine mixture (1 : 1, V/V, 2 ml) at room temperature for 1 hour to eliminate cyanoethyl group of phosphoric acid of the 3'-terminal group and the mixture was azeotropically distilled with pyridine for dehydration. The resulting freed dinucleotide was condensed with the thymidine on the polystyrene resin by reacting them at 40°C for 30 minutes in 0.2 ml of anhydrous pyridine in the presence of 2,4,6-trimethylbenzenesulfonyl-3-nitrotriazolide (30 mg). After washing with 2 ml of pyridine, the resin was treated with 1 ml of pyridineacetic anhydride mixture (9 : 1, V/V) containing 50 mg of dimethylaminopyridine, thereby acetylating 5'-hydroxyl group of unreacted thymidine. Subsequently, completely protected dinucleotides, GpAp, ApGp, TpTp, GpGp, TpCp and GpAp 25 mg each were successively reacted with the resulted resin in the same manner as above to form pentadecamer. After the last condensation operation, the resulting resin was washed with 2 ml of pyridine and then treated with 1 ml of 90% aqueous pyridine containing 0.5 M pyridinealdoxime and tetramethylguanidine at 40°C for 1 day to cleave the oligonucleotide off the resin. The oligonucleotide was then treated with 3 ml of 28% aqueous ammonia at 60°C for 4 hours to give the pentadecamer of which protective groups were completely eliminated except dimethoxytrityl group at the 5'-end. The pentadecamer was then purified by high performance liquid chromatography (HPLC) with reversed phase carriers (Cosmosil 5C18®; manufactured by Nakari Chemical) wherein the pentadecamer was eluted with linear gradient of acetonitrile in 0.1 M acetic acid-triethylamine buffer (pH 6.5). The eluate was then condensed to 50 µl under reduced pressure. Glacial acetic acid (final concentration 80%) was added to the condensate, and the mixture was reacted at room temperatures for 15 minutes for elimination of dimethoxytrityl group. The resulting mixture was then subjected to HPLC in the same manner as above to yield 10 OD260 units of the completely freed pentadecamer. In the same fashion, other oligonucleotides shown in Fig. 4 were synthesized.

Phosphorylation and ligation of oligonucleotides

As shown in Fig. 4, the human lysozyme gene was assembled from the 56 oligonucleotides prepared as above. The oligonucleotides were divided into 9 blocks, Blocks A through I as shown in Fig. 4. The oligonucleotides of each block were first ligated into a duplexe. The resulting duplexes were assembled into 3 fragments A-B-C, D-E-F and G-H-I, which were again ligated to form the human lysozyme gene. In this process, the oligonucleotides except the first (HLY-1) and the last fragment (HLY-56) were phosphorylated and then sets of the fragments were ligated. As a representative example of phosphorylation and ligation of the fragments, details of construction of the block A fragment of Fig. 4 are given below.

A mixture of the 8 fragments (HLY-2 to HLY-9), 2 µg each was incubated at 37°C for 60 minutes in 70 µl of a reaction medium containing 8.4 units of T4 polynucleotide kinase, 35 p mol of ($\gamma$-$^{32}$p) ATP (2,900 Ci/m mol), 1 mM spermidine. 50 mM dithiothreitol (DTT), 100 mM MgCl$_2$, 500 mM Tis-HCl (pH 7.5) and 1 mM EDTA. After the mixture was further incubated at 65°C for 10 minutes, it was ice-cooled. To the mixture, 2 µg of the fragment HLY-1 and 20 mM Tris-HCl (pH 7.5) were added followed by addition of 1 M Tris-HCl and 1 M MgCl$_2$ to adjust to MgCl$_2$ contents to 10mM. The mixture was incubated at 65°C for 10 minutes and allowed to stand at 37°C for 30 minutes and for further 20 minutes at room temperature. ATP and DTT were added to the mixture to bring their contents to 0.4 mM and 10 mM, respectively. After the mixture was incubated at 11°C for 10 minutes, T4 ligase 22 units was added to it and the mixture was incubated at 11°C for 12 hours to ligase the fragments. After the reaction was over, DNA was recovered by phenol-extraction and ethanol-precipitation and dissolved in 50 µl of TE buffer (10 mM Tris-HCl pH 7.5, 1 mM EDTA). which was stored at −20°C. In the same manner, other block fragments B through I were prepared from the sets of the oligonucleotides HLY-10 to 14, HLY-15 to 18, HLY-19 to 22. HLY-23 to 26, HLY-27 to 31, HLY-32 to 38, HLY-39 to 47 and HLY-48 to 56, respectively. The resulting reaction mixtures of the block A, B and C fragments 25 µl each were combined and DNA was recovered from the mixture by ethanol-precipitation. The DNA was reacted at 11°C for 12 hours in the same reaction medium as the one used for the ligation of the oligonucleotides of the fragment A. The fragment A-B-C was isolated from the mixture by 18% polyacrylamide gel electrophoresis. In the same manner, fragments D-E-F and G-H-I were prepared. These fragments A-B-C, D-E-F and G-H-I were combined and ligated by reacting them at 11°C for 17 hours in the same reaction medium as used above to give the human lysozyme gene.

For insertion of the human lysozyme gene into a vector, the reaction mixture was used as it was.

Cloning

Plasmid pBR322 10 µg was incubated at 37°C for 2 hours in 50 µl of a reaction medium containing 10mM Tris-HCl (pH 7.5), 50 mM NaCl, 10 mM MgCl$_2$ and 50 units of restriction enzyme Bam HI and DNA was recovered by phenol-extraction and ethanol-precipitation. The resulting DNA 3.5 µg and the reaction mixture containing the human lysozyme gene were combined and the mixture was reacted at 11°C for 13 hours in 200 µl of reaction medium containing 20 mM Tris-HCl pH 7.5, 10 mM MgCl$_2$, 0.4 mM ATP, 10 mM DTT and 13 units of T4 ligase. With the resulting mixture was transformed E. coli in the manner known in the field of the invention. The transformants resistant to ampicilline (40 µg/ml) and sensitive to tetracycline (10 µg/ml) were selected, from which DNA was retrieved.

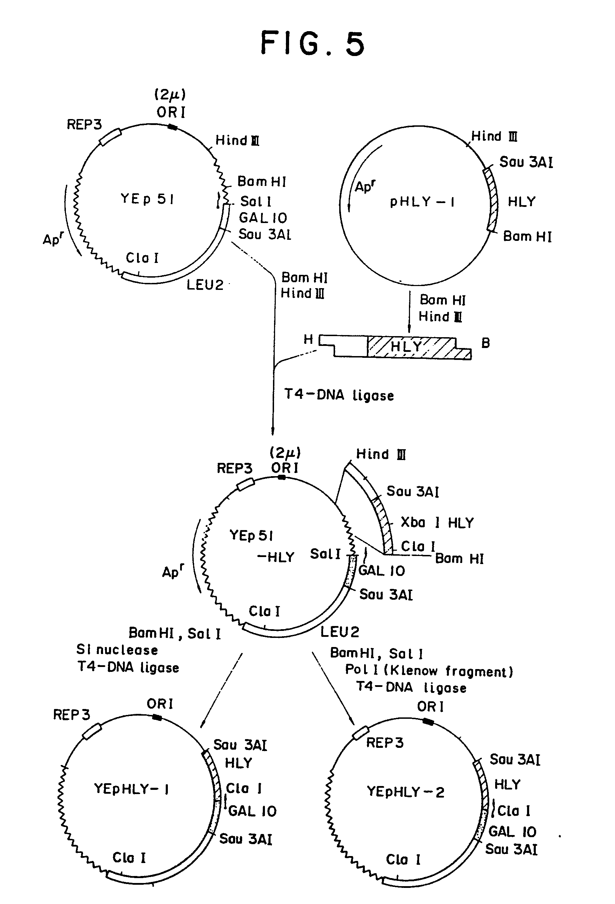

The restriction mapping of the DNA was carried out to select the transformants containing the recombinant plasmid into which the human lysozyme gene was inserted at Bam HI site of pBR322 in the orientation shown in Fig. 5 (Hereinafter, this plasmid is referred to as pHLY-1).

The recombinant plasmid pHLY-1 was digested with Sau 3AI, Bam HI/Hind III and Bam HI/Xba I and subjected to 8% polyacrylamide gel electrophoresis to give 418bp. 559bp and 205bp of fragments, respectively. The DNA sequences of these fragments were determined by Maxam-Gilbert method (Maxam et al. Proc. Natl. Acad. Sci. USA. 74 560 (1977)) to confirm that Sau 3AI fragment of 418bp of pHLY-1 has the designed DNA sequences shown in Fig 4.

Preparation of recombinant plasmid YEp51-HLY

Plasmid YEp51 (3 µg) was digested with Bam HI (15 units) and Hind III (12.5 units) by reacting it in 50 µl of Buffer I (10 mM Tris-HCl pH 7.5, 100 mM NaCl, 1 mM DTT) at 30°C for 4 hours.

Approximately 7Kb of DNA fragment containing GAL 10 promoter was isolated by 0.8% agarose gel electrophoresis. The DNA fragment (765bp) containing the human lysozyme gene was cleaved off the plasmid pHLY-1 by digesting the plasmid (30 μg) with Bam HI (140 units) and Hind III (115 units) in 70 μl of Buffer I at 37°C for 4 hours followed by electrophoresis on 5% polyacrylamide gel.

Thus isolated fragments (7Kb fragment: 0.35 μg; 765bp fragment: 1.2 μg) were ligated by reacting them at 15°C for 8 hours in 22 μl of Buffer II (66 mM TrisHCl pH 7.5, 10 mM MgCl$_2$, 10 mM DTT, 4 mM ATP) with T4 ligase (2.6 units).

With the reaction mixture, *E. coli* C 600 was transformed and ampicilline (80 μg/ml) resistant transformants were selected. DNA was isolated from the transformants and restriction mapping of the DNA was conducted to select the transformants having the recombinant plasmid YEp51-HLY of which restriction map was given in Fig. 5.

Preparation of expression plasmid YEpHLY-1

Approximately 7.5 Kbp of DNA fragment containing the human lysozyme gene was retrieved by digesting the plasmid YEp51-HLY (12 μg) with 40 units of Bam HI and 90 units of Sal I in 70 μl of Buffer I at 37°C for 5 hours followed by 0.7% agarose gel electrophoresis. The 7.5 Kb DNA fragment (2 μg) was reacted in 100 μl of reaction medium containing 250 mM sodium acetate pH 4.5, 1 mM ZnCl$_2$, 100 mM NaCl and 1.5 g/ml (final concentration) of SI nuclease (Sigma) at 8°C for 10 minutes. To the reaction medium was added 10 μl of 3M sodium acetate and the mixture was treated 3 times with phenol and DNA was recovered by ethanol precipitation. The DNA was ligated by reacting it at 18°C for 15 hours in Buffer II containing 2.6 units of T4 DNA ligase and 24 units of T4 RNA ligase. With the resulting mixture, *E. coli* C 600 was transformed and the transformants resistant to ampicilline (80 μg/ml) were selected.

Plasmid DNA was isolated from the transformants and restriction mapping of the DNA was carried out to select the transformants holding the recombinant plasmid YEpHLY-1 of which restriction mapping is shown in Fig. 5.

The plasmid YEpHLY-1 (80 μg) was reacted in 150 μl of reaction medium containing 10 mM Tris-HCl pH 7.5. 10 mM MgCl$_2$, 80 mM KCl, 1 mM DTT and 240 units of restriction enzyme Cla I at 37°C for 2 hours and DNA was collected by ethanol-precipitation. The resulting DNA (10 μg) was reacted at 37°C for 60 minutes in reaction medium containing 100 mM Tris-HCl pH 8.0 and 1 unit of bacterial alkaline phosphatase. NaCl was added to the reaction mixture to a final concentration of 100 mM and DNA was recovered by extracting with phenol for 5 times and precipitating the DNA with ethanol.

The DNA was reacted at 37°C for 45 minutes in 30 μl of reaction medium containing 50 mM Tris-HCl pH 7.6, 10 mM MgCl$_2$, 5 mM DTT, 0.1 mM spermidine, 0.1 mM EDTA, 60pmol (180Ci) of [γ-$^{32}$P] ATP (manufactured by New England Nuclease, 3.000 Ci/m mol) and 16 units of T4 polynucleotide kinase. By phenol-extraction followed by ethanol-precipitation, DNA was recovered.

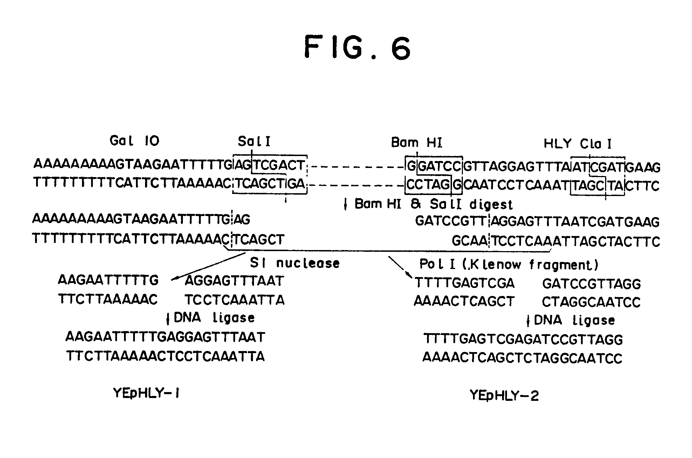

The DNA was digested with Eco RI (33 units) in 50 μl of Buffer I at 37°C for 2 hours and applied to 1% agarose gel electrophoresis to give 1.5 Kbp of DNA labled with $^{34}$P. DNA sequencing of the DNA was carried out according to Maxam-Gilbert method (Maxam et al. Proc. Natl. Acad. Sci. USA. *74* 560 (1977)) to confirm that it has the DNA sequences given in Fig. 6 and Fig. 2.

Preparation of expression Plasmid YEpHLY-2

The plasmid YEp51-HLY (12 μg) was digested with 40 units of Bam HI and 90 units of Sal I in 70 μl of Buffer I at 37°C for 5 hours and approximately 7.5 Kbp of DNA fragment containing the human lysozyme gene was isolated by 0.7% agarose gel electrophoresis.

The DNA fragment (1 μg) was reacted in 25 μl of reaction medium containing 50 mM Tris-HCl pH 7.2, 10 mM MgSO$_4$, 0.1 mM DTT, 50 μg/ml BSA, each 2 n mol of d NTP d ATP d CTP and d TTT 9 p mol of α-$^{32}$P. d CTP (Amersham, 800 Ci/m mol) and 1 unit of DNA polymerase (klenow fragment) at room temperature for 30 minutes. After addition of 2 μl of 250 mM ETDA, gel-filtration of the mixture with Sephadex G-50® was carried out and $^{32}$P-labeled DNA of high molecular fractions was recovered by phenol-extraction and ethanol-precipitation. The DNA was ligated by reacting it in 106 μl of reaction medium containing Buffer II, 10 units of T4 DNA ligase and 24 units of T4 DNA ligase at 18°C for 14 hours. *E. coli* C 600 was transformed with the reaction mixture and the ampicilline(80 μg/ml) resistant transformants were selected. Plasmid DNA was isolated from the transformants and the restriction mapping of the DNA was carried out to select the transformants containing the plasmid YEpHLY-2 which has the restriction mapping given in Fig. 5. The DNA sequencing of the plasmid YEpHLY-2 was conducted in the same manner as in the case of YEP HLY-1. Fig. 6 shows the DNA sequencing of the junction between GAL-10 promoter region and the human lysozyme gene.

Preparation of yeast transformants

*S. cerevisiae* KK4 was transformed with YEp51, YEpHLY-1 and YEpHLY-2, 5μg-each according to the Spheroplasts method (Hinnen et al. Proc. Natl. Acad. Sci. USA. *75* 1929 (1978)) and the transformants not requiring leucine were selected. The resulting transformants were named as *S. cerevisiae* KK4 (YEp51) *S. cerevisiae* KK4 (YEpHLY-1) (ATCC 20801) and *S. cerevisiae* KK4 (YEpHLY-2) (ATCC 20802), respectively.

Expression of human lysozyme

After the transformants, *S. cerevisiae* KK4 (YEp51), *S. cerevisiae* KK4 (YEpHLY-1) and *S. cerevisiae* KK4 (YEpHLY-2) were grown at 37°C in 5 ml of minimal medium containing all the necessary amino acids except leucine and methionine (final concentration; 20 to 375 mg/l), adenine sulfate (final concentration; 20 mg/l) and 2% galactose as a carbon source (Sherman et al. Method in Yeast Genetics P.62 Cold Spring Harbour (1982)) until reaching their middle logarithmic phase, 50 μCi of $^{35}$S-methionine (Amersham) was added to the medium and the mixture was further incubated at 30°C for 1 hour with shaking. The cells were collected by centrifugation at 5,000 rpm for 5 minutes and incubated at 30°C for 30 minutes in a buffer containing 0.65 mg of Zymolyase 100T and 2M sorbitol.

The cells were again collected by centrifugation at 5,000 rpm for 5 minutes, and they are suspended in a lytic buffer (10 mM $Na_3PO_4$ pH 7.2, 1 mM phenylmethanesulfonyl chloride, 50 mM EDTA, 0.9% NaCl, 10% Triton X-100, 0.5% deoxycholate and 0.5% SDS) to extract protein. To the protein extract solution (360 μl), rabbit anti-human lysozyme antibody (The Green Cross Corporation) was added and incubated at 10°C for 16 hours. The mixture was further incubated at room temperature for 60 minutes after 300 μl of STDS buffer (10 mM Tris-HCl pH 8.0, 1% Triton X-100, 0.5% sodium deoxycholate, 0.5% SDS) and 90 μl of protein A-Sepharose CL-4B (Pharmacia) suspension in STDS (10% V/V) were added.

The mixture was then laid on 300 μl of STDS buffer containing 10% sucrose and centrifuged at 12,000 rpm for 1 minute. The precipitate was washed 4 times with 200 μl of STDS buffer. The precipitate was suspended in 50 μl of a buffer (62.5 mM Tris-Hcl pH 6.8, 10% glycerol, 2.5% SDS, 2mM2-mercaptoethanol, 5 mM EDTA, 0.001% BPB) and heated at 100°C for 5 minutes. The mixture was applied to SDS-polyacrylamide (15%)-gel electrophoresis and fluorography was carried out to analyse the protein. In the resulting electrophoretic patterns, 14.7K dalton bands corresponding to the human lysozyme were observed with the lanes of the proteins extracted from *S. cerevisiae* KK4 (YEpHLY-1) and KK4 (YEpHLY-2) containing the human lysozyme gene but not with the lane of *S. cerevisiae* KK4 (YEp51) containing no human lysozyme gene. Thus the expression of the human lysozyme gene was confirmed.

**Claims**

1. A recombinant expression plasmid for yeasts comprising a yeast expression vector DNA containing GAL 10 promoter and a synthetic human lysozyme gene having the DNA sequence given in Fig. 2 inserted into said yeast expression vector at its insertion site and linked to said promoter through a 5'-noncoding region containing a DNA sequence which is

AAGAATTTTTGAGGAGTTTAAT or TTTTGAGTCGAGATCCGTTAGG

TTCTTAAAAACTCCTCAAATTA AAAACTCAGCTCTAGGCAATCC

2. A recombinant expression plasmid according to claim 1 wherein the yeast expression vector DNA containing GAL 10 promoter is YEp51.

3. A recombinant plasmid YEpHLY-1 (ATCC 20801).

4. A recombinant plasmid YEpHLY-2 (ATCC 20802).

5. Transformant yeast capable of producing human lysozyme which is produced by transforming yeast with a recombinant expression plasmid as claimed in any one of the preceding claims.

6. Transformant yeast according to claim 5 which originally belongs to *Saccharomyces cerevisiae*.

7. Transformant yeast according to claim 6 which is originated from *Saccharomyces cerevisiae* KK4 strain.

8. *Saccharomyces cerevisiae* KK4 (YEpHLY-1) (ATCC 20801).

9. *Saccharomyces cerevisiae* KK4 (YEpHLY-2) (ATCC 20802).

10. A process for producing human lysozyme which comprises culturing a transformant yeast as claimed in any one of claims 5 to 9 in a suitable culture medium and recovering human lysozyme from the cultured transformant yeast.

**Patentansprüche**

1. Rekombinantes Expressionsplasmid für Hefen mit einer Hefeexpressionsvektor-DNS, enthaltend den GAL 10 Promotor und ein synthetisches humanes Lysozymgen mit der in Fig. 2 angegebenen DNS-Sequenz, das in den Hefeexpressionsvektor an seiner Insertionsstelle eingesetzt und mit dem Promotor durch eine 5'-nichtkodierende Region, welche die DNS-Sequenz

AAGAATTTTTGAGGAGTTTAAT oder TTTTGAGTCGAGATCCGTTAGG

TTCTTAAAAACTCCTCAAATTA AAAACTCAGCTCTAGGCAATCC

enthält, verbunden ist.

2. Rekombinantes Expressionsplasmid nach Anspruch 1, worin die Hefeexpressionsvektor-DNS, enthaltend den GAL 10 promotor, YEp51 ist.

3. Rekombinantes Plasmid YEpHLY-1 (ATCC 20801).

4. Rekombinantes Plasmid YEpHLY-2 (ATCC 20802).

5. Transformierte, zur Herstellung von humanem Lysozym fähige Hefe, die durch die Transformierung von Hefe mit einem rekombinanten Expressionsplasmid, wie es in einem der vorhergehenden Ansprüche beansprucht ist, hergestellt ist.

6. Transformierte Hefe nach Anspruch 5, die ursprünglich zu *Saccharomyces cerevisiae* gehört.

7. Transformierte Hefe nach Anspruch 6, die in dem *Saccharomyces cerevisiae* KK4-Stamm ihren Ursprung hat.

8. *Saccharomyces cerevisiae* KK4 (YEpHLY-1) (ATCC 20 801).

9. *Saccharomyces cerevisiae* KK4 (YEpHLY-2) (ATCC 20 802).

10. Verfahren zur Herstellung von humanem Lysozym, das die Kultivierung einer transformierten Hefe, wie sie in einem der Ansprüche 5 bis 9 beansprucht wird, in einem geeigneten Kulturmedium und die Gewinnung von humanem Lysozym aus der kultivierten transformierten Hefe umfaßt.

**Revendications**

1. Plasmide d'expression recombinant pour des levures, comprenant un vecteur ADN d'expression de levure contenant un promoteur GAL 10 et un gène de lysozyme humain synthétique présentant la séquence ADN représentée à la figure 2, insérée dans ledit vecteur d'expression de levure, à son site d'insertion, et liée audit promoteur par une région de non-codage 5' contenant une séquence ADN qui est

AAGAATTTTTGAGGAGTTTAAT ou TTTTGAGTCGAGATCCGTTAGG

TTCTTAAAAACTCCTCAAATTA AAAACTCAGCTCTAGGCAATCC

2. Plasmide d'expression recombinant selon la revendication 1, dans lequel le vecteur ADN d'expression de levure contenant le promoteur GAL 10 est YEp51.

3. Plasmide recombinant YEpHLY-1 (ATCC 20801).

4. Plasmide recombinant YEpHLY-2 (ATCC 20802).

5. Levure transformante capable de produire du lysozyme humain qui est obtenu par transformation de la levure avec un plasmide d'expression recombinant selon l'une quelconque des revendications précédentes.

6. Levure transformante selon la revendication 5, appartenant originairement au *Saccharomyces cerevisiae*.

7. Levure transformante selon la revendication 6, provenant de la souche KK4 de *Saccharomyces cerevisiae*.

8. *Saccharomyces cerevisiae* KK4 (YEpHLY-1) (ATCC 20801).

9. *Saccharomyces cerevisiae* KK4 (YEpHLY-2) (ATCC 20802).

10. Procédé de production de lysozyme humain, consistant à cultiver une levure transformante selon l'une quelconque des revendications 5 à 9, dans un milieu de culture approprié, et à récupérer le lysozyme humain à partir de la levure transformante cultivée.

# FIG. 1

```
Lys - Val - Phe - Glu - Arg -
 1
Cys - Glu - Leu - Ala - Arg -
                          10
Thr - Leu - Lys - Arg - Leu -
Gly - Met - Asp - Gly - Tyr -
                          20
Arg - Gly - Ile - Ser - Leu -
 21
Ala - Asn - Trp - Met - Cys -
                          30
Leu - Ala - Lys - Trp - Glu -
Ser - Gly - Tyr - Asn - Thr -
                          40
Arg - Ala - Thr - Asn - Tyr -
 41
Asn - Ala - Gly - Asp - Arg -
                          50
Ser - Thr - Asp - Tyr - Gly -
Ile - Phe - Gln - Ile - Asn -
                          60
Ser - Arg - Tyr - Trp - Cys -
 61
Asn - Asp - Gly - Lys - Thr -
                          70
Pro - Gly - Ala - Val - Asn -
Ala - Cys - His - Leu - Ser -
                          80
Cys - Ser - Ala - Leu - Leu -
 81
Gln - Asp - Asn - Ile - Ala -
                          90
Asp - Ala - Val - Ala - Cys -
Ala - Lys - Arg - Val - Val -
                          100
Arg - Asp - Pro - Gln - Gly -
 101
Ile - Arg - Ala - Trp - Val -
                          110
Ala - Trp - Arg - Asn - Arg -
Cys - Gln - Asn - Arg - Asp -
                          120
Val - Arg - Gln - Tyr - Val -
 121
Gln - Gly - Cys - Gly - Val
                          130
```

1

# FIG. 2A

```
AAG    GTT    TTC    GAA    CGT
TTC    CAA    AAG    CTT    GCA


TGT    GAA    TTG    GCC    AGA
ACA    CTT    AAC    CGG    TCT


ACT    TTG    AAG    AGA    TTG
TGA    AAC    TTC    TCT    AAC


GGT    ATG    GAC    GGT    TAC
CCA    TAC    CTG    CCA    ATG


CGT    GGT    ATC    TCT    TTG
GCA    CCA    TAG    AGA    AAC


GCT    AAC    TGG    ATG    TGT
CGA    TTG    ACC    TAC    ACA


TTG    GCC    AAG    TGG    GAA
AAC    CGG    TTC    ACC    CTT


TCT    GGT    TAC    AAC    ACT
AGA    CCA    ATG    TTG    TGA


AGA    GCT    ACT    AAC    TAC
TCT    CGA    TGA    TTG    ATG


AAC    GCC    GGT    GAC    CGT
TTG    CGG    CCA    CTG    GCA


TCT    ACT    GAC    TAC    GGT
AGA    TGA    CTG    ATG    CCA
```

## FIG. 2

| FIG. 2A |
| FIG. 2B |
| FIG. 2C |

# FIG. 2B

```
ATC   TTC   CAA   ATT   AAC
TAG   AAG   GTT   TAA   TTG


TCT   AGA   TAC   TGG   TGT
AGA   TCT   ATG   ACC   ACA


AAC   GAC   GGT   AAG   ACT
TTG   CTG   CCA   TTC   TGA


CCA   GGC   GCC   GTT   AAC
GGT   CCG   CGG   CAA   TTG


GCC   TGT   CAC   TTG   TCT
CGG   ACA   GTG   AAC   AGA


TGT   TCT   GCT   TTG   TTG
ACA   AGA   CGA   AAC   AAC


CAA   GAC   AAC   ATC   GCT
GTT   CTG   TTG   TAG   CGA


GAC   GCC   GTT   GCC   TGT
CTG   CGG   CAA   CGG   ACA


GCT   AAG   AGA   GTC   GTT
CGA   TTC   TCT   CAG   CAA


AGA   GAC   CCA   CAA   GGT
TCT   CTG   GGT   GTT   CCA
```

# FIG. 2C

```
ATC   AGA   GCT   TGG   GTC
TAG   TCT   CGA   ACC   CAG

GCT   TGG   CGT   AAC   AGA
CGA   ACC   GCA   TTG   TCT

TGT   CAA   AAC   AGA   GAC
ACA   GTT   TTG   TCT   CTG

GTC   AGA   CAA   TAC   GTT
CAG   TCT   GTT   ATG   CAA

CAA   GGT   TGT   GGT   GTC
GTT   CCA   ACA   CCA   CAG
```

# FIG. 3A

GATCCGTTAGGAGTTTAATCG ATG

  GCAATCCTCAAATTAGC TAC

AAG   GTT   TTC   GAA   CGT

TTC   CAA   AAG   CTT   GCA

TGT   GAA   TTG   GCC   AGA

ACA   CTT   AAC   CGG   TCT

ACT   TTG   AAG   AGA   TTG

TGA   AAC   TTC   TCT   AAC

GGT   ATG   GAC   GGT   TAC

CCA   TAC   CTG   CCA   ATG

CGT   GGT   ATC   TCT   TTG

GCA   CCA   TAG   AGA   AAC

GCT   AAC   TGG   ATG   TGT

CGA   TTG   ACC   TAC   ACA

TTG   GCC   AAG   TGG   GAA

AAC   CGG   TTC   ACC   CTT

TCT   GGT   TAC   AAC   ACT

AGA   CCA   ATG   TTG   TGA

## FIG. 3

FIG. 3A

FIG. 3B

FIG. 3C

# FIG. 3B

```
AGA   GCT   ACT   AAC   TAC
TCT   CGA   TGA   TTG   ATG


AAC   GCC   GGT   GAC   CGT
TTG   CGG   CCA   CTG   GCA


TCT   ACT   GAC   TAC · GGT
AGA   TGA   CTG   ATG   CCA


ATC   TTC   CAA   ATT   AAC
TAG   AAG   GTT   TAA   TTG


TCT   AGA   TAC   TGG   TGT
AGA   TCT   ATG   ACC   ACA


AAC   GAC   GGT   AAG   ACT
TTG   CTG   CCA   TTC   TGA


CCA   GGC·  GCC   GTT   AAC
GGT   CCG   CGG   CAA   TTG


GCC   TGT   CAC   TTG   TCT
CGG   ACA   GTG   AAC   AGA


TGT   TCT   GCT   TTG   TTG
ACA   AGA   CGA   AAC   AAC


CAA   GAC   AAC   ATC   GCT
GTT   CTG   TTG   TAG   CGA
```

# FIG. 3C

```
GAC   GCC   GTT   GCC   TGT
CTG   CGG   CAA   CGG   ACA


GCT   AAG   AGA   GTC   GTT
CGA   TTC   TCT   CAG   CAA


AGA   GAC   CCA   CAA   GGT
TCT   CTG   GGT   GTT   CCA


ATC   AGA   GCT   TGG   GTC
TAG   TCT   CGA   ACC   CAG


GCT   TGG   CGT   AAC   AGA
CGA   ACC   GCA   TTG   TCT


TGT   CAA   AAC   AGA   GAC
ACA   GTT   TTG   TCT   CTG


GTC   AGA   CAA   TAC   GTT
CAG   TCT   GTT   ATG   CAA


CAA   GGT   TGT   GGT   GTC
GTT   CCA   ACA   CCA   CAG


TAA   T
ATT   ACT   AG
```

# FIG. 4A

```
        f   1    2    3    4    5    6    7    8    9   10   11   12   13   14   15   16   17
       Met  Lys  Val  Phe  Glu  Arg  Cys  Glu  Leu  Ala  Arg  Thr  Leu  Lys  Arg  Leu  Gly  Met
  ───HLY-1─────── ─────HLY-3─────── ────────HLY-5──────── ────────HLY-7──────── ────────HLY-9───────
  GATCCGTTAGGAGTTTAATCG ATG AAG GTT TTC GAA CGT TGT GAA TTG GCC AGA ACT TTG AAG AGA TTG GGT ATG

  ────GCAATCCTCAAATTAGC TAC TTC CAA AAG CTT GCA ACA CTT AAC CGG TCT TGA AAC TTC TCT AA
  BamHI              ClaI              TaqI            BalI          MboⅡ
  Sau3AI             TaqI                              HaeⅢ
  MboI    ── HLY-2 ─────────── ────HLY-4───────── ────HLY-6──────── ────HLY-8──────
          ─────────────────────────────────────── A ──────────────────────────────────────

     15   16   17   18   19   20   21   22   23   24   25   26   27   28   29   30
     Leu  Gly  Met  Asp  Gly  Tyr  Arg  Gly  Ile  Ser  Leu  Ala  Asn  Trp  Met  Cys
           ──────HLY-11────── ─────HLY-13───────
           GAC GGT TAC CGT GGT ATC TCT TTG GCT AAC
       C CCA TAC CTG CCA ATG GCA CCA TAG AGA AAC CGA TTG ACC TCA AC
                   BstEⅡ
     ──────HLY-10─────────── ─────HLY-12──────── ────HLY-14───────
     ───────────────────────────── B ─────────────────────────

     28   29   30   31   32   33   34   35   36   37   38   39   40
     Trp  Met  Cys  Leu  Ala  Lys  Trp  Glu  Ser  Gly  Tyr  Asn  Thr
     ──────HLY-15────── ──────HLY-17──────
     TGG ATG TGT TTG GCC AAG TGG GAA TCT GGT
           A AAC CGG TTC ACC CTT AGA CCA ATG TTG TG
                 BalI         HinfI
             HaeⅢ
         ──────HLY-16────── ──────HLY-18──────
     ─────────────────────── C ───────────────────────
```

# FIG. 4B

```
    38   39   40    41   42   43   44   45   46   47   48   49   50
   Tyr  Asn  Thr   Arg  Ala  Thr  Asn  Tyr  Asn  Ala  Gly  Asp  Arg

   ——— HLY-19 ———        ——— HLY-21 ———

   TAC  AAC  ACT  AGA  GCT  ACT  AAC  TAC  AAC  GCC
                A   TCT  CGA  TGA  TTG  ATG  TTG  CGG  CCA  CTG  GC
                     AluI                               BstEⅡ
                                                   MspI
                                                   HpaⅡ

   ——— HLY-20 ———————————        ——— HLY-22 ———

   —————————————————————— D ——————————————————————
```

```
    48   49   50    51   52   53   54   55   56   57   58   59   60   61
   Gly  Asp  Arg   Ser  Thr  Asp  Tyr  Gly  Ile  Phe  Gln  Ile  Asn  Ser

   ——— HLY-23 ———        ——— HLY-25 ———

   GGT  GAC  CGT  TCT  ACT  GAC  TAC  GGT  ATC  TTC
                A   AGA  TGA  CTG  ATG  CCA  TAG  AAG  GTT  TAA  TTG  A
   HphI                                    MboⅡ
              ——— HLY-24 ———————        ——— HLY-26 ———

   —————————————————————— E ——————————————————————
```

```
    58   59   60    61   62   63   64   65   66   67   68   69   70   71   72
   Gln  Ile  Asn   Ser  Arg  Tyr  Trp  Cys  Asn  Asp  Gly  Lys  Thr  Pro  Gly

   ——— HLY-27 ———————        ——— HLY-29 ———————        ——— HLY-31 ———

   CAA  ATT  AAC  TCT  AGA  TAC  TGG  TGT  AAC  GAC  GGT  AAG  ACT  CCA  GGC
                GA   TCT  ATG  ACC  ACA  TTG  CTG  CCA  TTC  TG
                XbaI                                    HinfⅠ
                                                         BstNⅠ
                                                         EcoRⅡ
              ——— HLY-28 ———————        ——— HLY-30 ———

   —————————————————————— F ——————————————————————
```

FIG. 4

| FIG. 4A |
| FIG. 4B |
| FIG. 4C |

EP 0 208 472 B1

# FIG. 4C

```
 70  71  72  73  74  75  76  77  78  79  80  81  82  83  84  85  86  87  88  89  90
Thr Pro Gly Ala Val Asn Ala Cys His Leu Ser Cys Ser Ala Leu Leu Gln Asp Asn Ile Ala

        ——————HLY-33——————        ————HLY-35—————        ——HLY-37—————
        GCC GTT AAC GCC TGT CAC TTG TCT TGT TCT GCT TTG TTG CAA GAC
A GGT CCG CGG CAA TTG CGG ACA GTG AAC AGA ACA AGA CGA AAC AAC GTT CTG TTG TAG CG
       BbeI    HpaI
       NarI    HincII

   ———— HLY-32 —————————      ———HLY-34—————————      ———HLY-36——————————      ——HLY-38—————————

   ————————————————————————————————————— G —————————————————————————————————————


 88  89  90  91  92  93  94  95  96  97  98  99 100 101 102 103 104 105 106 107 108 109 110 111 112
Asn Ile Ala Asp Ala Val Ala Cys Ala Lys Arg Val Val Arg Asp Pro Gln Gly Ile Arg Ala Trp Val Ala Trp

   ——— HLY-39 —————————      —— HLY-41——————————      ——— HLY-43 ——————————      —— HLY-45—————————      ——— HLY-47——————————
AAC ATC GCT GAC GCC GTT GCC TGT GCT AAG AGA GTC GTT AGA GAC CCA CAA GGT ATC AGA GCT TGG GTC GCT TGG
        A CTG CGG CAA CGG ACA CGA TTC TCT CAG CAA TCT CTG GGT GTT CCA TAG TCT CGA ACC CA
          HgaI                      DdeI   HinfI                              AluI

      ——— HLY-40 —————————      ——HLY-42——————————      ——— HLY-44——————————      ——HLY-46—————————

   ————————————————————————————————————— H —————————————————————————————————————


110 111 112 113 114 115 116 117 118 119 120 121 122 123 124 125 126 127 128 129 130
Val Ala Trp Arg Asn Arg Cys Gln Asn Arg Asp Val Arg Gln Tyr Val Gln Gly Cys Gly Val Ter Ter

        —————HLY-49——————        ——— HLY-51 —————        —— HLY-53 ——————        —— HLY-55 ——————
        CGT AAC AGA TGT CAA AAC AGA GAC GTC AGA CAA TAC GTT CAA GGT TGT GGT GTC TAA T
    G CGA ACC GCA TTG TCT ACA GTT TTG TCT CTG CAG TCT GTT ATG CAA GTT CCA ACA CCA CAG ATT ACT AG
                                                                                      Sau3AI
                                                                                      MboI

   ——— HLY-48 —————————      ——— HLY-50—————————      ——— HLY-52 ——————————      ——HLY-54—————————      ——HLY-56———

   ————————————————————————————————————— I —————————————————————————————————————
```

# FIG. 5

EP 0 208 472 B1

# FIG. 6

```
         Gal IO              Sal I                    Bam HI              HLY Cla I
AAAAAAAAAAGTAAGAATTTTTG|AG|TCGACT|- - - - - - - - -|G|GATCC|GTTAGGAGTTTA|AT|CGAT|GAAG
TTTTTTTTTTCATTCTTAAAAAC|TCAGCT|GA|- - - - - - - - -|CCTAG|G|CAATCCTCAAAT|TAGC|TA|CTTC
                                                   | Bam HI & Sal I digest
AAAAAAAAAAGTAAGAATTTTTTG|AG                             GATCCGTT|AGGAGTTTAATCGATGAAG
TTTTTTTTTTCATTCTTAAAAAC|TCAGCT                              GCAA|TCCTCAAATTAGCTACTTC
                              SI nuclease              Po L I (Klenow fragment)
    AAGAATTTTTG    AGGAGTTTAAT                         TTTTGAGTCGA      GATCCGTTAGG
    TTCTTAAAAAC    TCCTCAAATTA                         AAAACTCAGCT      CTAGGCAATCC
              |DNA ligase                                          |DNA ligase
    AAGAATTTTTGAGGAGTTTAAT                           TTTTGAGTCGAGATCCGTTAGG
    TTCTTAAAAACTCCTCAAATTA                           AAAACTCAGCTCTAGGCAATCC

              YEpHLY-I                                          YEpHLY-2
```